# EUROPEAN PATENT SPECIFICATION

(11) **EP 0 351 853 B1**
(45) Date of publication and mention of the grant of the patent: **13.04.1994**
(21) Application number: 89113350.6
(22) Date of filing: 20.07.1989
(51) Int. Cl.: C12P 19/38, C07H 19/06

(54) **Method of manufacturing trifluorothymidine**
Verfahren zur Herstellung von Trifluorthymidin
Méthode de fabrication de trifluorothymidine

(30) Priority: 22.07.1988 JP 181742/88
(43) Date of publication of application: 24.01.1990
(73) Proprietor: YUKI GOSEI KOGYO CO., LTD., Chuo-ku Tokyo (JP); Japan Tobacco Inc., Minato-Ku Tokyo 105 (JP)
(72) Inventor: Sugimoto, Takahiro c/o Yuki Gosei Kogyo Co., Ltd., Tokyo (JP); Sunaga, Yonosuke c/o Yuki Gosei Kogyo Co., Ltd., Tokyo (JP); Hayashi, Yoshinori c/o Yuki Gosei Kogyo Co., Ltd., Tokyo (JP); Shiba, Takamitsu c/o Yuki Gosei Kogyo Co., Ltd., Tokyo (JP); Katsuyama, Akio c/o Japan Tobacco Inc., Yokohama-shi (JP); Mikami, Yoichi c/o Japan Tobacco Inc., Tokyo (JP)
(74) Representative: Popp, Eugen, Dr.

(56) References cited:
- CHEMICAL ABSTRACTS, vol. 96, no. 3, 18th January 1982, page 330, abstract no.18680p, Columbus, Ohio, US; & JP-A-81 102 794
- AGRIC. BIOL. CHEM., vol. 49, no. 11, 1985, pages 3239-3246, Tokyo, JP; T.UTAGAWA et al.: "Properties of nucleoside phosphorylase from Enterobacteraerogens"

## Description

The present invention relates to a method of manufacturing trifluorothymidine by utilizing an enzyme reaction and, more particularly, to a method of manufacturing trifluorothymidine by a base exchange reaction or the like of a nucleoside by using an enzyme.

Trifluorothymidine as a compound to be manufactured by the present invention is a kind of deoxyriboside of trifluoromethyluracil. This compound has antiviral properties at a low concentration and is a material suitable for therapies of herpetic diseases such as keratitis.

A conventional method of manufacturing pyrimidine ribonucleosides and pyrimidine deoxyribonucleosides is also known wherein a nucleoside is used as a ribose or deoxyribose donor and is reacted with a pyrimidine base in the presence of a bacterium to perform a base exchange reaction using an enzyme (Published Unexamined Japanese Patent Application No. 56-102794). According to this method, a strain belonging to genus Escherichia, Erwinia, Klebsiella, Bacterium, Enterobacter, Aeromonas, Serratia, Proteus, Xanthomonas, or Citorobacter is used. The reaction is performed in an aqueous solution at 50 to 65°C.

Agric. Biol. Chem. 49(11) 3239-3246, 1985 describes properties of nucleoside phosphorylase from Enterobacter derogenes. The reactions are performed at 60-65°C.

As described above, since trifluorothymidine is a kind of deoxyriboside of trifluoromethyluracil, in principle, trifluorothymidine can be manufactured by employing the conventional method described above. However, an example using a whole body of a bacterium belonging particularly to genus Serratia is not described in the above patent application. It was also found that the manufacture of trifluorothymidine by the above method using trifluoromethyluracil as a material base resulted in a very low yield.

It is an object of the present invention to provide a method of manufacturing trifluorothymidine at a high yield.

A method according to the present invention includes the steps of:
causing trifluoromethyluracil and a deoxyribose donor to react with each other in a solution containing a bacterium belonging to genus Serratia in a temperature range of 30 to 45°C, thereby producing trifluorothymidine; and
recovering the trifluorothymidine from the solution.

The present invention also comprises a method which includes the steps of:
preparing a given enzyme which is active in a reaction between trifluoromethyluracil and a deoxyribose donor and obtained from a bacterium belonging to genus Serratia, or an immobilized enzyme obtained by immobilizing the given enzyme;
causing a solution containing the trifluoromethyluracil and the deoxyribose donor to contact with the given enzyme or the immobilized enzyme in a temperature range of 30 to 45°C, thereby producing trifluorothymidine; and
recovering the trifluorothymidine from the solution.

According to the present invention, a bacterium belonging to genus Serratia, or the enzyme or the immobilized enzyme which is active in the above reaction and obtained from a cultured bacterium belonging to genus Serratia is used to continuously obtain trifluorothymidine at a high yield from the trifluoromethyluracil and the deoxyribose donor under moderate reaction conditions.

The present inventor made extensive studies on a method of manufacturing trifluorothymidine from trifluoromethyluracil and a deoxyribose donor on the basis of a base exchange reaction using a bacterium and achieved to provide a high yield by appropriately selecting a bacterium belonging to genus Serratia and a moderate temperature condition in the range of 30 to 45°C.

Of enzymes obtained from cultured bacteria belonging to genus Serratia, when a given enzyme which is active in the reaction or an immobilized enzyme which is obtained by immobilizing the given enzyme was used, trifluorothymidine was obtained at a high yield in the same manner as the case using the whole bacterial body according to the findings of the present inventors. In addition, when the immobilized enzyme is used, trifluorothymidine can be continuously obtained.

The present invention will be described in detail below.

Examples of bacteria belonging to genus Serratia used in the present invention are Serratia marcescens IFO3052, Serratia marcescens IFO3046, Serratia marcescens IFO3735, Serratia liquefaciens IFO12979, and Serratia liquefaciens ATCC11367. Of these examples, Serratia marcescens IFO3052 has an excellent effect. These bacteria can be obtained by, e.g., the following method. Strains of the above bacteria are cultured in normal culture media containing a carbon source such as glucose, maltose, blackstrap molasses, and an acidic-or enzymatic-hydrolysate of starch; a nitrogen source such as ammonium sulfate, ammonium chloride, and urea; an organic nutrient source such as an yeast extract, malt extract, peptone, and meat extract; and inorganic ions such as phosphate, magnesium, potassium, zinc, iron, and manganese. The suspensions can be directly used after cultivation. However, bacteria separated from the culture solutions by centrifugal separation, or a bacterial body broken by an ultrasonic wave or the like may be used in place of the suspensions. In this case, an amount of the bacteria or the broken bacterial bodies may be 0.1 to 40, preferably 10 to 40 mg/mℓ (wet bacteria) in buffer solution.

A supernatant obtained by centrifugal separation of the broken bacterial body, an active fraction obtained by salting-out of the supernatant, or an acetone-treated bacterium can be used as an enzyme of the present invention directly or after purification. Of these enzymatic materials, the supernatant obtained by centrifugal separation of the broken bacterial body is most effective. In this case, an amount of these enzymes may be the same as that obtainable from 0.1 to 40, preferably 10 to 40 mg/mℓ bacteria or broken bacterial bodies in buffer solution.

These enzymes can be immobilized. An immobilization method of these enzymes may be a method of adsorbing the enzyme on an ion exchange resin, or a method of including and immobilizing the enzyme by a gel such as calcium alginate, or a gel crosslinked by using glutaraldehyde. Of these methods, an adsorption method using a weak anion exchange resin is most preferable. An amount of the immobilized enzyme mad be decided by reaction conditions. For example, the amount may be equal to that obtainable from 0.1 to 40, preferably 10 to 40 mg/mℓ bacteria or broken bacterial bodies in buffer solution.

Examples of the deoxyribose donor used in the present invention are 2'-deoxyribonucleoside such as 2'-deoxyadenosine, 2'-deoxyinosine, 2'-deoxyguanosine, thymidine and 2'-deoxyuridine, and 2-deoxy-α-D-ribose-1-phosphoric acid. The content of such a deoxyribose donor is 1 to 10 times in mol. and preferably 2 to 5 times in mol. with respect to trifluoromethyluracil.

A reaction is generally performed in an aqueous solution. When the deoxyribose donor is 2'-deoxyribonucleoside, 1 to 50 mmol. of phosphoric acid are preferably added to the aqueous solution.

The reaction temperature falls within the range of 30 to 45°C because the yield is decreased at high temperatures. Since trifluoromethyluracil as the starting material and trifluorothymidine as the product of the present invention are chemically unstable, a moderate temperature condition is preferred.

The reaction time preferably falls within the range of 3 to 20 hours. A long reaction time is not preferable since the produced trifluorothymidine is unstable. After the reaction is completed, the whole bacterial body or enzyme is separated, and the produced trifluorothymidine is isolated and purified by resin adsorption or crystallization.

When the reaction is performed using an immobilized enzyme, it is filled in a column, and a substrate aqueous solution in which trifluoromethyluracil, the deoxyribose donor, and phosphoric acid, added as needed, are dissolved is passed through the column. The concentration of the substrate aqueous solution is determined by an amount of trifluoromethyluracil. The content of trifluoromethyluracil is generally 0.3 wt% or less. A passing rate is given as a dilution rate of 10 h⁻¹ or less and preferably 3 h⁻¹ or less. The unit of the dilution rate represents a ratio of the volume of the solution passed through the column to the volume of the column per hour.

The present invention will be described in detail by way of its examples.

### Example 1

100 mℓ of a culture solution containing 1 g/dℓ of glucose, 1 g/dℓ of peptone, 0.5 g/dℓ of yeast extract, and 0.5 g/dℓ of salt (pH: 7.0) were charged in a 500-mℓ Erlenmeyer flask. After the culture solution was sterilized in an autoclave, Serratia marcescens IFO3052 was inoculated in the culture solution once with a platinum loop and was cultured at 30°C for 24 hours. After the culture, cultured bacteria were separated from the culture solution by centrifugal separation.

The separated bacteria were washed with a physiological saline and was suspended in a 5 mM phosphate buffer solution (pH: 7.0) (25 mg wet bacteria/mℓ). 0.1 g/dℓ of trifluoromethyluracil and 0.5 g/dℓ of thymidine were added to this suspension and the mixture was reacted at 45°C for 6 hours. After the reaction, the resultant solution was analyzed by a high-performance liquid chromatography. An amount of trifluorothymidine produced in the above reaction was 89 mg/dℓ. A yield of trifluorothymidine from trifluoromethyluracil was 54.1%.

### Example 2

6 ℓ of a culture solution as in Example 1 were charged in a 10-ℓ jar-type fermenter, and Serratia marcescens IFO3052 was inoculated in the culture solution and cultured at 30°C for 24 hours. 126 mg/dℓ of trifluorothymidine were obtained when subsequent operations were performed following the same procedures as in Example 1. A yield of trifluorothymidine from trifluoromethyluracil was 76.6%.

### Example 3

72 mg/dℓ of trifluorothymidine were obtained upon performing operations following the same procedures as in Example 1 except that 2'-deoxyguanosine was used in place of thymidine of Example 1. A yield of trifluorothymidine from trifluoromethyluracil was 43.8%.

### Example 4

73 mg/dℓ of trifluorothymidine were obtained upon performing operations following the same procedures as in Example 1 except that 2'-deoxyadenosine was used in place of thymidine of Example 1. A yield of trifluorothymidine from trifluoromethyluracil was 44.4%.

### Example 5

Products were obtained following the same procedures as in Example 1 except that the reaction temperatures were 30°C, 40°C, 50°C, and 60°C. Yields of the trifluorothymidine from trifluoromethyluracil in the reactions at the above temperatures were summarized in Table 1.

**Table 1**

| Reaction Temperature (°C) | Yield (%) |
|---|---|
| 30 | 23.2 |
| 40 | 54.1 |
| 50 | 15.3 |
| 60 | 1.5 |

### Example 6

Bacteria treated as in Example 1 was suspended (25 mg wet bacteria/mℓ) in a 50-mM tris-hydrochloride buffer solution (pH: 7.5). 0.1 g/dℓ of trifluoromethyluracil and 0.5 g/dℓ of 2-deoxy-α-D-ribose-1-phosphoric acid were added to the solution, and the resultant mixture was reacted at temperatures of 30°C, 40°C, 50°C, and 60°C for 16 hours. After the reaction was completed, the resultant solution was analyzed following the same procedures as in Example 1. Yields of trifluorothymidine from trifluoromethyluracil at the respective reaction temperatures are summarized in Table 2.

**Table 2**

| Reaction Temperature (°C) | Yield (%) |
|---|---|
| 30 | 21.8 |
| 40 | 49.8 |
| 50 | 12.6 |
| 60 | 0.7 |

### Example 7

One liter of a culture solution (pH: 7.0) containing 10 g/ℓ of glucose, 10 g/ℓ of peptone, 5 g/ℓ of yeast extract, and 5 g/ℓ of salt was charged in a 3-ℓ flask and was sterilized in an autoclave. Serratia marcescens IFO3052 was subjected to shaking culture in 100 mℓ of a culture solution having the same composition as the above culture solution in a 500-mℓ flask at 30°C for 16 hours. 5 mℓ of this culture solution were inoculated in the sterilized culture solution and were cultured at 30°C for 16 hours. After the culture was completed, cultured bacteria were separated by centrifugal separation. 8 g of the wet bacteria were suspended in 16 mℓ of a 15 mM phosphate buffer solution (pH: 7.0), and 5 mM of ethylenediaminetetraacetic acid were added thereto to break the bacterial body with an ultrasonic wave while the solution was cooled by ice. The broken bacteria were separated by centrifugal separation to obtain a supernatant, and the supernatant was used as a coarse enzyme solution.

20 mℓ of a weak anion exchange resin (TOYOPEARL: DEAE 650M) were filled in a column having a diameter of 16 mm, and 20 mℓ of the above coarse enzyme solution were brought into contact with the ion exchange resin to sufficiently adsorb the enzyme on the anion exchange resin. Thereafter, the anion exchange resin was sufficiently washed with 3.7 mM of phosphate buffer solution (pH: 7.0).

A substrate solution obtained by dissolving substrates (7.5 g/ℓ of thymidine and 2.0 g/ℓ of trifluoromethyluracil) in a 3.7 mM phosphate buffer solution (pH: 7.0) was continuously passed through the column at a flow rate of 10 mℓ/hr for 517 hours. The resultant effluent was analyzed by a high-performance liquid chromatography. A yield of trifluorothymidine from the charged trifluoromethyluracil was 65%.

## Claims

1. A method of manufacturing trifluorothymidine, comprising the steps of:
causing trifluoromethyluracil and a deoxyribose donor to react with each other in a solution containing a bacterium belonging to genus Serratia in a temperature range of 30 to 45°C, thereby producing trifluorothymidine; and
recovering the trifluorothymidine from the solution.

2. A method according to claim 1, characterized in that the deoxyribose donor is a material selected from the group consisting of 2'-deoxyadenosine, 2'-deoxyinosine, 2'-deoxyguanosine, thymidine, 2'-deoxyuridine, and 2-deoxy-α-D-ribose-1-phosphoric acid.

3. A method according to claim 2, characterized in that the bacterium belonging to genus Serratia is Serratia marcescens or Serratia liquefaciens.

4. A method of manufacturing trifluorothymidine, comprising the steps of:
preparing a given enzyme which is active in a reaction between trifluoromethyluracil and a deoxyribose donor and obtained from a bacterium belonging to genus Serratia, or an immobilized enzyme obtained by immobilizing the given enzyme;
causing a solution containing the trifluoromethyluracil and the deoxyribose donor to contact with the given enzyme or the immobilized enzyme in a temperature range of 30 to 45°C, thereby producing trifluorothymidine; and
recovering the trifluorothymidine from the solution.

5. A method according to claim 4, characterized in that the deoxyribose donor is a material selected from the group consisting of 2'-deoxyadenosine, 2'-deoxyinosine, 2'-deoxyguanosine, thymidine, 2'-deoxyuridine, and 2-deoxy-α-D-ribose-1-phosphoric acid.

6. A method according to claim 5, characterized in that the bacterium belonging to genus Serratia is Serratia marcescens or Serratia liquefaciens, and the enzyme is a material selected from the group consisting of a supernatant obtained by centrifugal separation of broken bacterial body, an active fraction obtained by salting-out of the supernatant, an acetone-treated bacterium, and a purified material thereof.

## Patentansprüche

1. Verfahren zum Herstellen von Trifluorthymidin, das die folgenden Schritte aufweist:
Bewirken, daß Trifluormethyluracil und ein Desoxyribosedonor in einer Lösung, die eine der Gattung Serratia angehörende Bakterie enthält, in einem Temperaturbereich von 30-45 °C miteinander reagieren, wodurch Trifluorthymidin erzeugt wird; und
Gewinnen des Trifluorthymidins aus der Lösung.

2. Verfahren nach Anspruch 1, dadurch gekennzeichnet, daß der Desoxyribosedonor ein Material ist, das aus der Gruppe ausgewählt ist, die aus 2'-Desoxyadenosin, 2'-Desoxyinosin, 2'-Desoxyguanosin, Thymidin, 2'-Desoxyuridin und 2-Desoxy-α-D-ribose-1-phosphorsäure besteht.

3. Verfahren nach Anspruch 2, dadurch gekennzeichnet, daß die der Gattung Serratia angehörende Bakterie Serratia marcescens oder Serratia liquefaciens ist.

4. Verfahren zum Herstellen von Trifluorthymidin, das die folgenden Schritte aufweist:
Bereitstellen eines gegebenen Enzyms, das bei einer Reaktion zwischen Trifluormethyluracil und einem Desoxyribosedonor wirksam und von einer der Gattung Serratia angehörenden Bakterie erhalten ist oder eines immobilisierten Enzyms, das durch Immobilisieren des gegebenen Enzyms erhalten ist;
In-Kontakt-Bringen einer Lösung, die Trifluormethyluracil und den Desoxyribosedonor enthält, mit dem gegebenen Enzym oder dem immobilisierten Enzym in einem Temperaturbereich von 30-45 °C, wodurch Trifluorthymidin erzeugt wird; und
Gewinnen des Trifluorthymidins aus der Lösung.

5. Verfahren nach Anspruch 4, dadurch gekennzeichnet, daß der Desoxyribosedonor ein Material ist, das aus der Gruppe ausgewählt ist, die aus 2'-Desoxyadenosin, 2'-Desoxyinosin, 2'-Desoxyguanosin, Thymidin, 2'-Desoxyuridin und 2-Desoxy-α-D-ribose-1-phosphorsäure besteht.

6. Verfahren nach Anspruch 5, dadurch gekennzeichnet, daß die der Gattung Serratia angehörende Bakterie Serratia marcescens oder Serratia liquefaciens ist und das Enzym ein Material ist, das aus der Gruppe ausgewählt ist, die aus einem Überstand, der durch Trennung eines aufgebrochenen Bakterienkörpers durch Zentrifugation erhalten ist, einer aktiven Fraktion, die durch Aussalzen des Überstands erhalten ist, einer acetonbehandelten Bakterie und einem gereinigten Material davon besteht.

## Revendications

1. Procédé de préparation de trifluorothymidine, qui comprend les étapes consistant à :
- faire réagir l'un avec l'autre du trifluorométhyluracile et un donneur de désoxyribose, dans une solution contenant une bactérie appartenant au genre Serratia, à une température comprise dans l'intervalle allant de 30 à 45°C, pour produire ainsi de la trifluorothymidine, et
- récupérer la trifluorothymidine à partir de la solution.

2. Procédé selon la revendication 1, **caractérisé** en ce que le donneur de désoxyribose est un produit choisi parmi la 2ʼ-désoxyadénosine, la 2'-désoxyinosine, la 2'-désoxyguanosine, la thymidine, la 2'-désoxyuridine et le 2-désoxy-α-D-ribose-1-phosphate.

3. Procédé selon la revendication 2, **caractérisé** en ce que la bactérie appartenant au genre Serratia est Serratia marcescens ou Serratia liquefaciens.

4. Procédé de préparation de la trifluorothymidine, comprenant les étapes consistant à :
- préparer une enzyme donnée qui est active dans la réaction entre le trifluorométhyluracile et un donneur de désoxyribose et qui est obtenue à partir d'une bactérie appartenant au genre Serratia, ou une enzyme immobilisée, obtenue par immobilisation de l'enzyme donnée,
- mettre une solution contenant le trifluorométhyluracile et le donneur de désoxyribose en contact avec l'enzyme donnée ou l'enzyme immobilisée, à une température comprise dans l'intervalle allant de 30 à 45°C, pour produire ainsi de la trifluorothymidine, et
- récupérer la trifluorothymidine à partir de la solution.

5. Procédé selon la revendication 4, **caractérisé** en ce que le donneur de désoxyribose est un produit choisi parmi la 2'-désoxyadénosine, la 2'-désoxyinosine, la 2'-désoxyguanosine, la thymidine, la 2'-désoxyuridine et le 2-désoxy-α-D-ribose-1-phosphate.

6. Procédé selon la revendication 5, **caractérisé** en ce que la bactérie appartenant au genre Serratia est Serratia marcescens ou Serratia liquefaciens, et l'enzyme est un produit choisi parmi le surnageant obtenu par séparation par centrifugation de corps bactériens brisés, une fraction active obtenue par relargage du surnageant, une bactérie traitée par l'acétone, et un produit purifié obtenu à partir des précédents.
